# EUROPEAN PATENT APPLICATION

(11) **EP 4 148 744 A1**
(43) Date of publication of application: **15.03.2023**
(21) Application number: 21821925.1
(22) Date of filing: 21.04.2021
(51) Int. Cl.: G16H 40/63, A61B 5/02, A61B 5/026, A61B 5/0285

(54) **METHOD AND DEVICE FOR GENERATING ARTERIOSCLEROSIS DETECTION INTERFACE, TERMINAL, AND SYSTEM**

(30) Priority: 08.06.2020 CN 202010514082
(71) Applicant: Huawei Technologies Co., Ltd., Longgang District, Shenzhen, Guangdong 518129 (CN)
(72) Inventor: WANG, Wei, Shenzhen, Guangdong 518129 (CN); QIU, Lingzhi, Shenzhen, Guangdong 518129 (CN); ZHANG, Jie, Shenzhen, Guangdong 518129 (CN)
(74) Representative: Pfenning, Meinig & Partner mbB
(86) International application number: PCT/CN2021/088716
(87) International publication number: WO 2021/249034

(57) **Abstract**

A method and an apparatus for generating an arteriosclerosis detection interface, a terminal, and a system are provided. The method includes: obtaining a type of an arteriosclerosis detector in use; generating a detection interface and determining an artery detection position corresponding to the type of the arteriosclerosis detector; and displaying the artery detection position on the detection interface. On the arteriosclerosis detection interface, the artery detection position of the arteriosclerosis detector may be displayed to a user, to prompt the user which artery segment is the artery detection position in this detection. Even if the user is a non-professional, the user can intuitively determine an artery segment area detected during arteriosclerosis measurement.

## Description

### TECHNICAL FIELD

The present invention relates to the field of display control technologies, and in particular, to a method and an apparatus for generating an arteriosclerosis detection interface, a terminal, and a system.

### BACKGROUND

With the rapid development of the national economy, people's material life has been greatly enriched. However, in modern times, due to increasing life pressure, disordered work and rest, unreasonable dietary structure, and the like, an increasing quantity of people suffer from cardiovascular diseases. According to the World Health Organization, about 12 million people die from cardiovascular diseases every year in the world, accounting for 25% of the world's total deaths. It is estimated that deaths of cardiovascular diseases will account for 30% by 2020. Cardiovascular diseases have become a dire threat to people's health. Arteriosclerosis is the pathological basis of many cardiovascular diseases, and may cause coronary heart disease, cerebral stroke, lower extremity obliterans, and other diseases. Arteriosclerosis is a generic term for a series of arterial pathological changes, such as thickening, deformation, hardening of the arterial wall, fat deposition on the endarterium, and plaque and thrombus formation. Evidence in recent years shows that functional changes in arterial wall elasticity occur long before changes in the arterial structure. Therefore, early screening and active intervention for abnormal arterial function have unique significance in cardiovascular upstream prevention and control.

Left ventricular contraction of the heart pulsates blood into the aorta, expands the aortic wall to produce pressure pulse waves, and conducts the pressure pulse waves along the vascular wall to peripheral arteries at a specific speed. A conduction velocity of a pulse wave in an artery is a PWV (Pulse wave velocity, pulse wave velocity). Theoretically, a PWV can be measured between any artery segments on a human body. PWVs of different artery segments reflect different degrees of arteriosclerosis, but clinically, a PWV of the carotid femoral artery is used as the gold standard for evaluating arteriosclerosis.

After an existing detection device detects a user, when only a value of an AoPWV (Aortic Pulse Wave Velocity, aortic pulse wave velocity) is provided, although the detection result indicates that the device detects an aortic PWV, a non-professional does not clearly know which artery segment the aortic is specifically referred to. When only a user-defined MOCA Index (Montreal Cognitive Assessment Index, Montreal cognitive assessment index) value is provided, the detection result does not clearly indicate which artery segment is measured in terms of a degree of arteriosclerosis. In addition, only a value is provided, which is not intuitive. Similarly, when only a PWV value is provided after the detection, the detection result does not clearly indicate which artery segment is measured in terms of a PWV value, which is not intuitive.

### SUMMARY

Embodiments of the present invention provide a method and an apparatus for generating an arteriosclerosis detection interface, a terminal, and a system, to obtain a type of an arteriosclerosis detector used when a user performs arteriosclerosis measurement, determine, based on the type of the arteriosclerosis detector, an artery detection position corresponding to the type of the arteriosclerosis detector, and further display, when generating a detection interface, the artery detection position on the generated detection interface. Different arteriosclerosis detectors detect different artery segments, and an artery detection position of an arteriosclerosis detector may be displayed to the user, to prompt the user which artery segment is the artery detection position in this detection. Even if the user is a non-professional, the user can intuitively determine an artery segment area detected during arteriosclerosis measurement.

An embodiment of the present invention provides a method for generating an arteriosclerosis detection interface. The method includes:
obtaining a type of an arteriosclerosis detector in use;
generating a detection interface and determining an artery detection position corresponding to the type of the arteriosclerosis detector; and
displaying the artery detection position on the detection interface.

Further, the detection interface includes a human artery distribution diagram, and the displaying the artery detection position on the detection interface includes: highlighting an artery segment at the artery detection position in the human artery distribution diagram.

Further, before the generating a detection interface and determining an artery detection position corresponding to the type of the arteriosclerosis detector, the method further includes:
obtaining gender information of a to-be-detected user; and
determining a type of the human artery distribution diagram based on the gender information of the to-be-detected user.

Further, the highlighting an artery segment at the artery detection position on the detection interface includes:
enabling the artery segment at the artery detection position in the human artery distribution diagram to blink; or
displaying the artery segment at the artery detection position in the human artery distribution diagram, and hiding remaining artery segments.

Further, the method further includes:
obtaining a detection result from the arteriosclerosis detector; and
generating a detection result interface based on the detection result.

The detection result interface includes the human artery distribution diagram, and the detection result pops up at the artery detection position in the human artery distribution diagram.

Further, the method further includes:
after the detection result pops up, generating prompt information for viewing an aortic arteriosclerosis degree at a heart position in the human artery distribution diagram; and
popping up aortic arteriosclerosis degree information at the heart position in the human artery distribution diagram according to a tap-to-view instruction of a user.

The aortic arteriosclerosis degree information includes an aortic pulse wave velocity and an aortic condition analysis result.

Further, the method further includes:
generating prompt information for viewing personal information of a user; and
expanding and displaying the personal information of the user according to a tap-to-view instruction of the user.

The personal information of the user includes one or more of the following:
name, gender, age, contact information, and historical medical treatment data.

An embodiment of the present invention further provides an apparatus for generating an arteriosclerosis detection interface. The apparatus includes:
a processor and a memory, where the memory is configured to store at least one instruction, and when the instruction is loaded and executed by the processor, the method for generating an arteriosclerosis detection interface is implemented.

An embodiment of the present invention further provides a terminal. The terminal includes the apparatus for generating an arteriosclerosis detection interface.

An embodiment of the present invention further provides an arteriosclerosis detection system. The system includes:
an arteriosclerosis detector, configured to detect a pulse wave velocity of a user; and
the terminal.

Further, the arteriosclerosis detector includes one or more of the following:
a smart watch, a smart band, and a body fat scale.

According to the foregoing technical solution, a type of an arteriosclerosis detector in use is obtained, a detection interface is generated, an artery detection position corresponding to the type of the arteriosclerosis detector is determined, and the artery detection position is displayed on the detection interface, to intuitively display the artery detection position of the arteriosclerosis detector to a user, to prompt the user which artery segment is the artery detection position in this detection. Even if the user is a non-professional, the user can intuitively determine an artery segment area detected during arteriosclerosis measurement.

### BRIEF DESCRIPTION OF DRAWINGS

To describe the technical solutions in embodiments of the present invention or in the conventional technology more clearly, the following briefly describes the accompanying drawings for describing embodiments or the conventional technology. It is clear that the accompanying drawings in the following description show some embodiments of the present invention, and persons of ordinary skill in the art may still derive other drawings from these accompanying drawings without creative efforts.
FIG. 1 is a flowchart of a method for generating an arteriosclerosis detection interface according to an embodiment of the present invention;
FIG. 2A is a male human artery distribution diagram according to an embodiment of the present invention;
FIG. 2B is a female human artery distribution diagram according to an embodiment of the present invention;
FIG. 3A to FIG. 3E are schematic diagrams of detection interfaces according to an embodiment of the present invention;
FIG. 4A to FIG. 4C are schematic diagrams of detection result interfaces according to an embodiment of the present invention;
FIG. 5 is a schematic diagram of a structure of an apparatus for generating an arteriosclerosis detection interface according to an embodiment of the present invention; and
FIG. 6 is a schematic diagram of displaying a terminal-based interface according to an embodiment of the present invention.

### DESCRIPTION OF EMBODIMENTS

Terms used in implementations of this application are merely used to explain specific embodiments of this application, but are not intended to limit this application. Some embodiments provided in this application may be applied to speech recognition in the artificial intelligence (Artificial Intelligence, AI for short) field, are related to a natural language processing (Natural Language Processing, NLP for short) technology, and may be specifically applied to an application such as a voice assistant.

To make the objectives, technical solutions, and advantages of embodiments of the present invention clearer, the following clearly and completely describes the technical solutions in embodiments of the present invention with reference to the accompanying drawings in embodiments of the present invention. It is clear that the described embodiments are a part rather than all of embodiments of the present invention. All other embodiments obtained by persons of ordinary skill in the art based on embodiments of the present invention without creative efforts shall fall within the protection scope of the present invention.

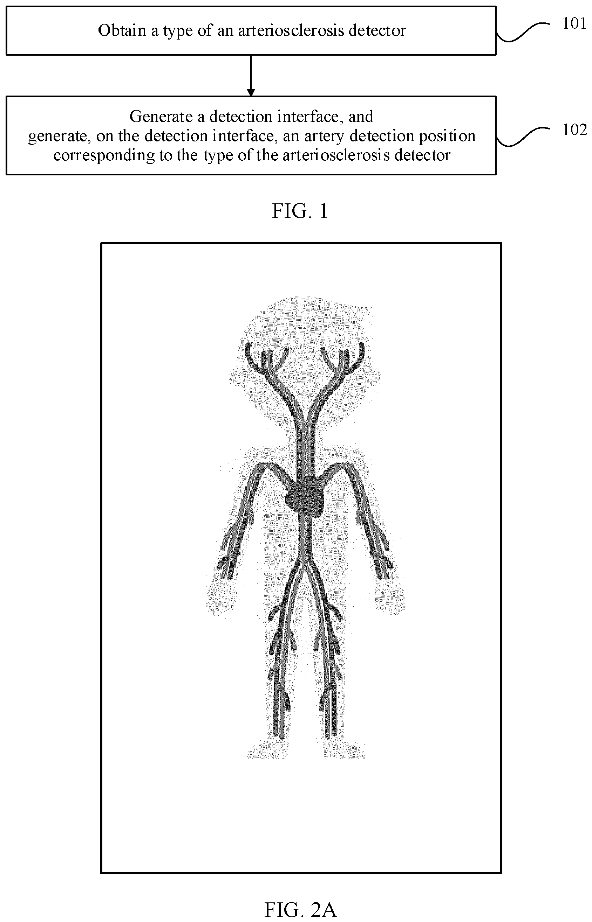

FIG. 1 is a flowchart of a method for generating an arteriosclerosis detection interface according to an embodiment of the present invention. As shown in FIG. 1, the method for generating an arteriosclerosis detection interface in this embodiment may specifically include the following steps.

Step 101: Obtain a type of an arteriosclerosis detector in use.

Step 102: Generate a detection interface and determine an artery detection position corresponding to the type of the arteriosclerosis detector, and display the artery detection position on the detection interface.

The following uses specific embodiments to describe in detail technical solutions of the method embodiments shown in FIG. 1 to FIG. 4C.

### (1) Preparation phase of user arteriosclerosis detection

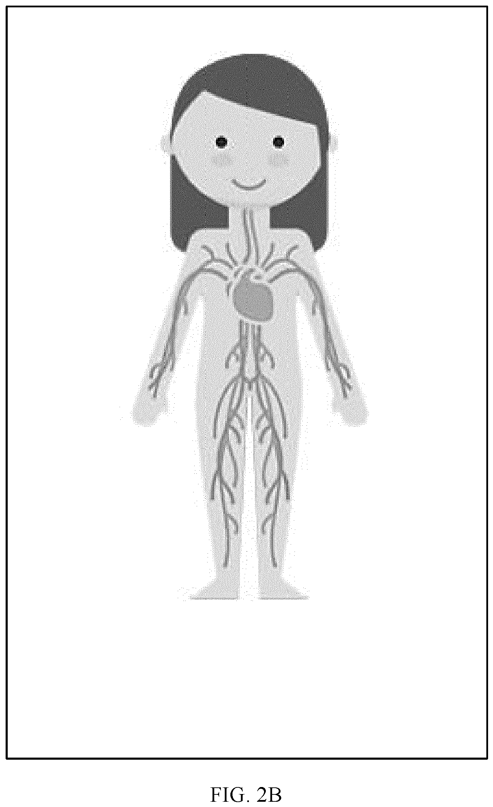

Obtain gender information and age information of a to-be-detected user based on personal information input by the to-be-detected user, determine, based on the gender information and age information of the to-be-detected user, a type of a human artery distribution diagram applicable to the gender and age of the to-be-detected user, extract a human artery distribution diagram of a corresponding gender type based on the determined type of the human artery distribution diagram, and generate an initial interface including the extracted human artery distribution diagram applicable to the to-be-detected user. Specifically, a plurality of human artery distribution diagrams (for example, a 2D human artery distribution diagram and a 3D human artery distribution diagram) may be prestored, and each human artery distribution diagram may be further classified, based on age groups, into human artery distribution diagrams applicable to the elderly, adults, adolescents, and infants, and each type of human artery distribution diagram includes a male version and a female version. For example, if a current to-be-detected user is a male user aged 20, a male human artery distribution diagram shown in FIG. 2A is generated; or if a current to-be-detected user is a female user aged 30, a female human artery distribution diagram shown in FIG. 2B is generated.

### (2) Phase of user arteriosclerosis detection

Obtain a type of an arteriosclerosis detector currently used by a user, generate a detection interface and determine an artery detection position corresponding to the type of the arteriosclerosis detector, and display the artery detection position on the detection interface. Specifically, because types of arteriosclerosis detectors used by the user are different, corresponding arteriosclerosis detection positions of various types of arteriosclerosis detectors are also different. For example, if the user wears a smart band to perform arteriosclerosis detection, the artery detection position is an artery segment from a sternum notch to a radial artery. If the user uses a body fat scale to perform arteriosclerosis detection, the artery detection position is an artery segment from the sternum notch to a toe. A type of a corresponding arteriosclerosis detector may be preset based on an arteriosclerosis detection position of an arteriosclerosis detector. For example, a type of an arteriosclerosis detector whose artery detection position is the artery segment from the sternum notch to the radial artery may be uniformly set to type A, and a type of an arteriosclerosis detector whose artery detection position is the artery segment from the sternum notch to the toe may be uniformly set to type B.

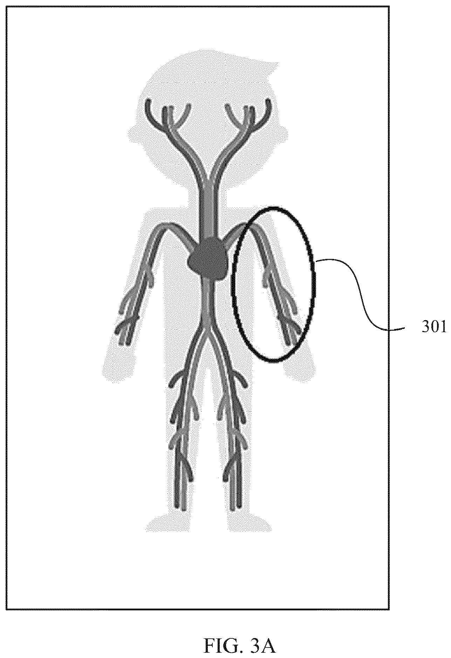

The generated detection interface may include the human artery distribution diagram, and the displaying the artery detection position on the detection interface may also be: highlighting an artery segment at the artery detection position in the human artery distribution diagram. Specifically, FIG. 3A shows a schematic diagram of a detection interface according to this embodiment of the present invention. As shown in FIG. 3A, when the arteriosclerosis detector used by the user is a smart band, in a process of performing arteriosclerosis detection, the user may obtain that a type of the arteriosclerosis detector in use is type A (that is, determine that the artery detection position is an artery segment 301 from the sternum notch to the radial artery). Then, the artery segment 301 from the sternum notch to the radial artery blinks in the human artery distribution diagram.

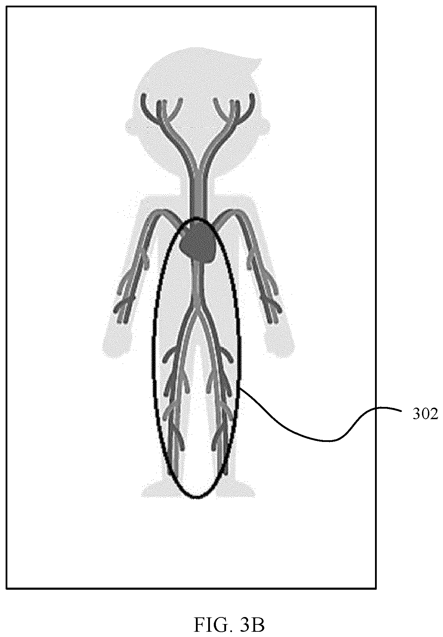

With different types of obtained arteriosclerosis detectors in use, artery segments blinking in the human artery distribution diagram are different. Specifically, FIG. 3B is a schematic diagram of another detection interface according to this embodiment of the present invention. As shown in FIG. 3B, when the arteriosclerosis detector used by the user is a body fat scale, in a process of performing arteriosclerosis detection, the user may obtain that a type of the arteriosclerosis detector in use is type B (that is, determine that the artery detection position is an artery segment 302 from the sternum notch to the toe). Then, the artery segment 302 from the sternum notch to the toe blinks in the human artery distribution diagram.

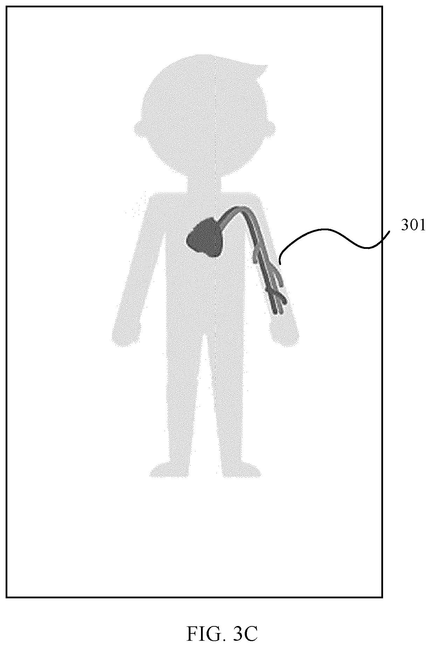

In this embodiment, highlighting an artery segment corresponding to the arteriosclerosis detector in the human artery distribution diagram may also be displaying the artery segment corresponding to the arteriosclerosis detector in the human artery distribution diagram, and hiding remaining artery segments. Specifically, FIG. 3C shows a schematic diagram of another detection interface according to this embodiment of the present invention. As shown in FIG. 3C, when the arteriosclerosis detector used by the user is the smart band, in the process of performing arteriosclerosis detection, the user may obtain that the type of the arteriosclerosis detector in use is type A (that is, determine that the artery detection position is the artery segment 301 from the sternum notch to the radial artery). Then, the artery segment 301 from the sternum notch to the radial artery is displayed in the human artery distribution diagram, and the remaining artery segments are hidden.

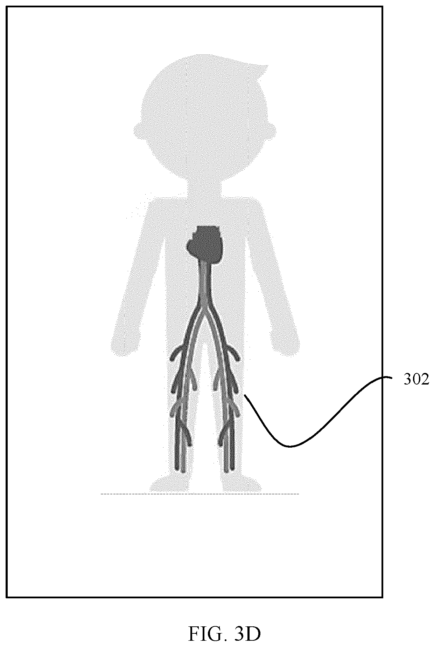

With different types of obtained arteriosclerosis detectors in use, artery segments displayed and hidden in the human artery distribution diagram are different. Specifically, FIG. 3D is a schematic diagram of another detection interface according to this embodiment of the present invention. As shown in FIG. 3D, when the arteriosclerosis detector used by the user is the body fat scale, in the process of performing arteriosclerosis detection, the user may obtain that the type of the arteriosclerosis detector in use is type B (that is, determine that the artery detection position is the artery segment 302 from the sternum notch to the toe). Then, the artery segment 302 from the sternum notch to the toe is displayed in the human artery distribution diagram, and the remaining artery segments are hidden.

When the user simultaneously uses two or more types of arteriosclerosis detectors to perform arteriosclerosis detection, to enable the user to distinguish a correspondence between each arteriosclerosis detector and a respective detection position, an identifier of a corresponding arteriosclerosis detector and a correspondence between an identifier of each arteriosclerosis detector and a corresponding detection position may be added near the highlighted display area in the human artery distribution diagram.

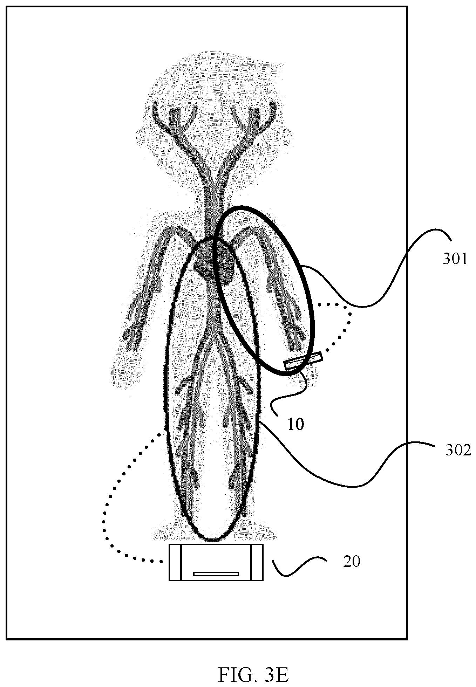

Specifically, FIG. 3E is a schematic diagram of another detection interface according to this embodiment of the present invention. As shown in FIG. 3E, when the user uses both the smart band and the body fat scale to perform arteriosclerosis detection, in the process of performing arteriosclerosis detection, the user may obtain that the type of the arteriosclerosis detector in use is type A+B (that is, determine that the artery detection position is the artery segment 301 from the sternum notch to the radial artery and the artery segment 302 from the sternum notch to the toe). Then, the artery segment 301 from the sternum notch to the radial artery and the artery segment 302 from the sternum notch to the toe simultaneously blink in the human artery distribution diagram. Further, an identifier 10 of the smart band is added near the artery segment 301 from the sternum notch to the radial artery, and an identifier 20 of the body fat scale is added near the artery segment 302 from the sternum notch to the toe. In addition, the artery segment 301 from the sternum notch to the radial artery and the identifier 10 of the smart band are connected by using a connection line of one color, and the artery segment 302 from the sternum notch to the toe and the identifier 20 of the body fat scale are connected by using a connection line of another color.

In this embodiment, when the user uses both the smart band and the body fat scale to perform arteriosclerosis detection, in the process of performing arteriosclerosis detection, the user obtains the type of the arteriosclerosis detector. For example, the type of the arteriosclerosis detector in use is type A+B. The artery segment 301 from the sternum notch to the radial artery and the artery segment 302 from the sternum notch to the toe may also be simultaneously displayed in the human artery distribution diagram, and the remaining artery segments are hidden. Further, the identifier 10 of the smart band is added near the artery segment 301 from the sternum notch to the radial artery, and the identifier 20 of the body fat scale is added near the artery segment 302 from the sternum notch to the toe. In addition, the artery segment 301 from the sternum notch to the radial artery and the identifier 10 of the smart band are connected by using a connection line of one color, and the artery segment 302 from the sternum notch to the toe and the identifier 20 of the body fat scale are connected by using a connection line of another color.

A manner of displaying the correspondence between each arteriosclerosis detector and the respective detection position may be implemented in another manner, and is not limited to a manner of performing connection by using connection lines of different colors.

### (3) Phase of displaying a user arteriosclerosis detection result

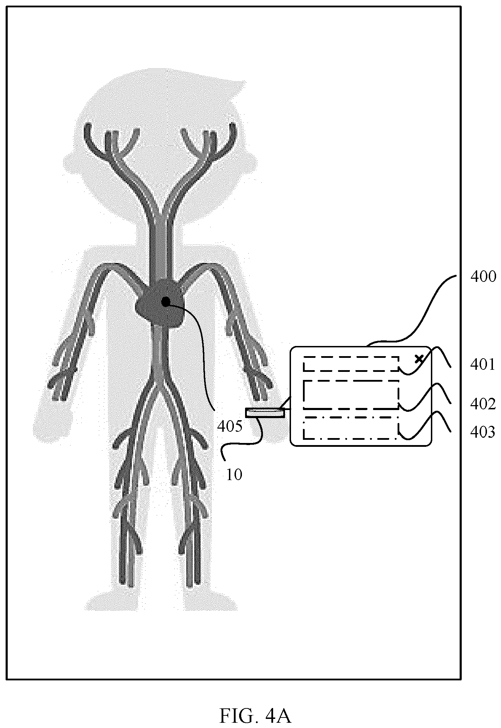

After the arteriosclerosis detection phase, a detection result provided by the arteriosclerosis detector is obtained, and a detection result interface is generated based on the detection result. The detection result interface may also include the human artery distribution diagram, and a display box 400 including the detection result 401 may pop up at a position that is in the human artery distribution diagram and that is corresponding to the type of the arteriosclerosis detector. Specifically, when the user performs arteriosclerosis detection by using the smart band, a detection result interface shown in FIG. 4A is generated. The detection result 401 provided by the smart band pops up at a corresponding wrist position (a wearing position of the smart band) in the human artery distribution diagram. For example, the detection result 401 may include a PWV: 8.2 m/s. A pop-up position of the detection result 401 may prompt the user of a detection position related to the detection result, that is, the pop-up position may prompt the user that this PWV mainly reflects a degree of arteriosclerosis of upper extremity.

To avoid a problem that the user cannot determine the artery detection position based on the pop-up position of the detection result 401, extended information about a text description 402 of the artery detection position may be further added to the display box 400 including the detection result 401. For example, the following information pops up at the corresponding wrist position in the human artery distribution diagram:
① detection result 401: "PWV: 8.2 m/s"; and
② the text description 402 of the artery detection position: "This PWV mainly reflects a degree of arteriosclerosis of upper extremity".

To provide a further service, the text description 402 and extended information of prompt information 403 for viewing an aortic arteriosclerosis degree may be further added to the display box 400 including the detection result 401. For example, the following information pops up at the corresponding wrist position in the human artery distribution diagram:
① detection result 401: "PWV: 8.2 m/s";
② the text description 402 of the artery detection position: "This PWV mainly reflects a degree of arteriosclerosis of upper extremity"; and
③ the prompt information 403 for viewing the aortic arteriosclerosis degree: "To know the aortic arteriosclerosis degree, tap the blinking point at the heart position in the figure".

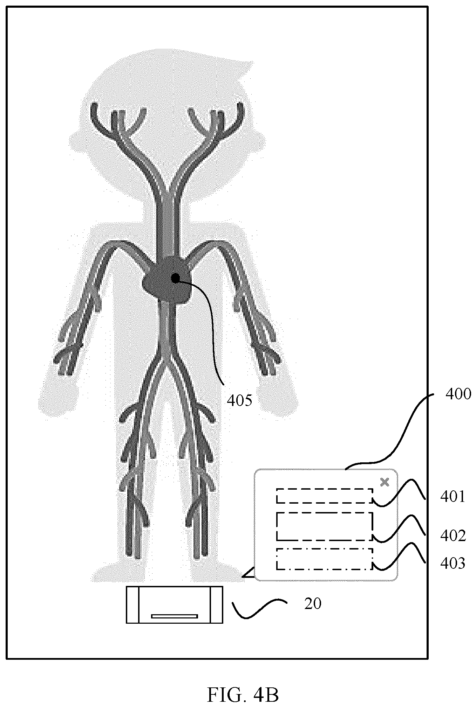

In this embodiment, when the user performs arteriosclerosis detection by using the body fat scale, a schematic diagram of a detection result interface shown in FIG. 4B is generated. The display box 400 including the detection result 401 pops up from a foot bottom position in the human artery distribution diagram. As shown in FIG. 4B, in addition to popping up the detection result 401, the display box 400 may include the text description 402 of the artery detection position and the extended information of the prompt information 403 for viewing the aortic arteriosclerosis degree. Details are similar to the foregoing manner of popping up the display box 400, and are not described again.

In the phase of displaying the user arteriosclerosis detection result, the human artery distribution diagram on the detection result interface may be displayed in a same manner as the human artery distribution diagram on the detection interface in the preparation phase of the user arteriosclerosis detection. Specifically, division is performed based on a quantity of arteriosclerosis detectors. When the quantity of arteriosclerosis detectors is 1, for example, when the display box 400 including the detection result provided by the smart band and the corresponding extended information pops up, the artery segment 301 from the sternum notch to the radial artery blinks, and when the display box 400 including the detection result provided by the body fat scale and the corresponding extended information pops up, the artery segment 302 from the sternum notch to the toe blinks. When the quantity of arteriosclerosis detectors is 2 or greater than 2, for example, when a display box including detection results provided by the smart band and the body fat scale and corresponding extended information pops up, the artery segment 301 from the sternum notch to the radial artery and the artery segment 302 from the sternum notch to the toe simultaneously blink, and identifiers (the identifier 10 of the smart band and the identifier 20 of the body fat scale) of corresponding arteriosclerosis detectors are added near respective blinking display areas.

In this embodiment, in the phase of displaying the user arteriosclerosis detection result, a manner of displaying the human artery distribution diagram on the detection interface may alternatively be: When the display box 400 including the detection result provided by the smart band and the corresponding extended information pops up, the artery segment 301 from the sternum notch to the radial artery is displayed, and the remaining artery segments are hidden. When the display box 400 including the detection result provided by the body fat scale and the corresponding extended information pops up, the artery segment 302 from the sternum notch to the toe is displayed, and the remaining artery segments are hidden. When the quantity of arteriosclerosis detectors is 2 or greater than 2, for example, when a display box 400 including detection results of the smart band and the body fat scale and corresponding extended information pops up, the artery segment 301 from the sternum notch to the radial artery and the artery segment 302 from the sternum notch to the toe are displayed simultaneously, the remaining artery segments are hidden, and the identifiers (the identifier 10 of the smart band and the identifier 20 of the body fat scale) of corresponding arteriosclerosis detectors are added near respective blinking display areas.

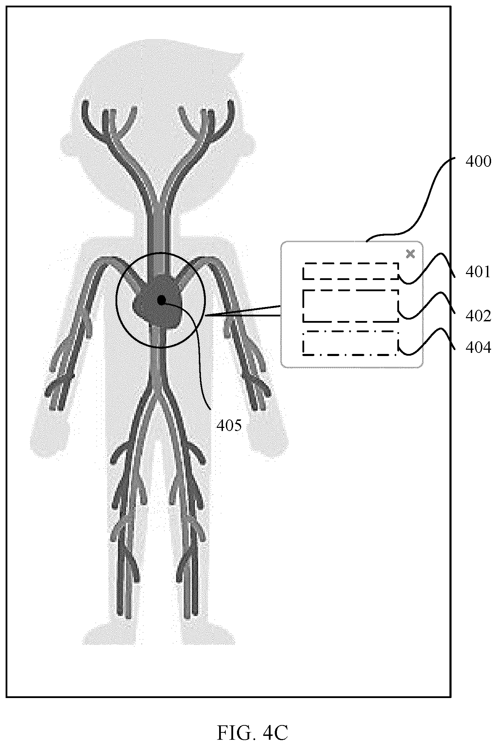

As shown in FIG. 4A or FIG. 4B, after the prompt information 403 for viewing the aortic arteriosclerosis degree is added to the display box 400 in which the detection result 401 is located, the prompt information for viewing the aortic arteriosclerosis degree may be further generated at the heart position in the human artery distribution diagram (for example, a blinking touch point 405 is provided at the heart position in the human artery distribution diagram), and aortic arteriosclerosis degree information pops up at the heart position in the human artery distribution diagram according to a tap-to-view instruction of the user. The aortic arteriosclerosis degree information includes: an aortic pulse wave velocity and an aortic condition analysis result. Specifically, the prompt information for viewing the aortic arteriosclerosis degree may be shown in the following manner: The blinking touch point 405 is provided at the heart position in the human artery distribution diagram, and the display box 400 including the aortic arteriosclerosis degree information shown in FIG. 4C pops up according to the tap-to-view instruction of the user. For example, the display box 400 includes:
① detection result 401: "PWV: 8.2 m/s";
② the text description 402 of the artery detection position: "This PWV mainly reflects an aortic arteriosclerosis degree"; and
③ an aortic condition analysis result 404: "Your aorta is in good condition. Please keep it up!"

In this embodiment, prompt information for viewing personal information of the user may be further generated, and the personal information of the user is expanded and displayed according to a tap-to-view instruction of the user. The personal information of the user includes one or more of the following: name, gender, age, contact information, and historical medical treatment data. It should be noted that the prompt information for viewing the personal information of the user may be generated in each phase (the preparation phase of the user arteriosclerosis detection, the phase of user arteriosclerosis detection, and the phase of displaying the user arteriosclerosis detection result).

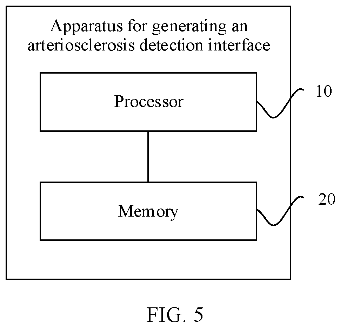

As shown in FIG. 5, an embodiment of the present invention further provides an apparatus for generating an arteriosclerosis detection interface. The apparatus may include a processor 10 and a memory 20. The memory 20 is configured to store at least one instruction. When the instruction is loaded and executed by the processor 10, the following method for generating an arteriosclerosis detection interface is implemented:
obtaining a type of an arteriosclerosis detector in use;
generating a detection interface and determining an artery detection position corresponding to the type of the arteriosclerosis detector; and
displaying the artery detection position on the detection interface.

Further, the detection interface includes a human artery distribution diagram, and the displaying the artery detection position on the detection interface includes: highlighting an artery segment at the artery detection position in the human artery distribution diagram.

Further, before the generating a detection interface and determining an artery detection position corresponding to the type of the arteriosclerosis detector, the method further includes:
obtaining gender information of a to-be-detected user; and
determining a type of the human artery distribution diagram based on the gender information of the to-be-detected user.

Further, the highlighting an artery segment at the artery detection position on the detection interface includes:
enabling the artery segment at the artery detection position in the human artery distribution diagram to blink; or
displaying the artery segment at the artery detection position in the human artery distribution diagram, and hiding remaining artery segments.

Further, the method further includes:
obtaining a detection result from the arteriosclerosis detector; and
generating a detection result interface based on the detection result.

The detection result interface includes the human artery distribution diagram, and the detection result pops up at the artery detection position in the human artery distribution diagram.

Further, the method further includes:
after the detection result pops up, generating prompt information for viewing an aortic arteriosclerosis degree at a heart position in the human artery distribution diagram; and
popping up aortic arteriosclerosis degree information at the heart position in the human artery distribution diagram according to a tap-to-view instruction of a user.

The aortic arteriosclerosis degree information includes an aortic pulse wave velocity and an aortic condition analysis result.

Further, the method further includes:
generating prompt information for viewing personal information of a user; and
expanding and displaying the personal information of the user according to a tap-to-view instruction of the user.

The personal information of the user includes one or more of the following:
name, gender, age, contact information, and historical medical treatment data.

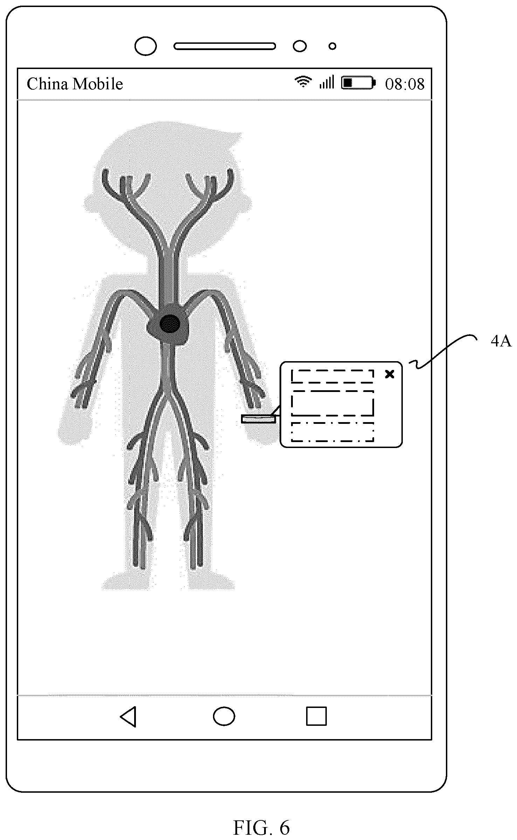

An embodiment of the present invention further provides a terminal. The terminal includes the apparatus for generating an arteriosclerosis detection interface. FIG. 6 is a schematic diagram of displaying a terminal-based interface according to an embodiment of the present invention. The terminal may display, on a display interface, any image involved in embodiments of FIG. 1 to FIG. 4C. As shown in FIG. 6, in a phase of displaying a user arteriosclerosis detection result, the terminal may display a corresponding detection result interface, to notify a user of corresponding result information of current arteriosclerosis detection.

It should be noted that the terminal involved in embodiments of the present invention may include but is not limited to a personal computer (Personal Computer, PC), a personal digital assistant (Personal Digital Assistant, PDA), a wireless handheld device, a tablet computer (Tablet Computer), a mobile phone, an MP3 player, an MP4 player, and the like.

It may be understood that the application may be an application program (nativeApp) installed on the terminal, or may be a web page program (webApp) of a browser on the terminal. This is not limited in this embodiment of the present invention.

An embodiment of the present invention further provides an arteriosclerosis detection system. The system in this embodiment includes: an arteriosclerosis detector, configured to detect a pulse wave velocity of a user; and the terminal. The arteriosclerosis detector includes one or more of the following: a smartwatch, a smart band, and a body fat scale. The system may execute the technical solution of the embodiment of the method for generating an arteriosclerosis detection interface. Implementation principles and technical effects thereof are similar. Details are not described herein again.

It may be clearly understood by persons skilled in the art that, for convenient and brief description, for a detailed working process of the foregoing system, apparatus, and unit, refer to a corresponding process in the foregoing method embodiments. Details are not described herein again.

In the several embodiments provided in the present invention, it should be understood that the disclosed system, apparatus, and method may be implemented in other manners. For example, the described apparatus embodiment is merely an example. For example, the division into the units is merely logical function division and may be other division in an actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions in embodiments.

In addition, function units in embodiments of the present invention may be integrated into one processing unit, or each of the units may exist alone physically, or two or more units may be integrated into one unit. The integrated unit may be implemented in a form of hardware, or may be implemented in a form of hardware in addition to a software function unit.

The integrated unit implemented in the form of a software function unit may be stored in a computer-readable storage medium. The software function unit is stored in a storage medium and includes several instructions for instructing a computer apparatus (which may be a personal computer, a server, or a network apparatus) or a processor (Processor) to perform some of the steps of the methods described in embodiments of the present invention. The foregoing storage medium includes: any medium that can store program code, such as a USB flash drive, a removable hard disk, a read-only memory (Read-Only Memory, ROM), a random access memory (Random Access Memory, RAM), a magnetic disk, or an optical disc.

The foregoing descriptions are merely example embodiments of the present invention, but are not intended to limit the present invention. Any modification, equivalent replacement, or improvement made without departing from the spirit and principle of the present invention shall fall within the protection scope of the present invention.

Finally, it should be noted that the foregoing embodiments are merely intended to describe the technical solutions of the present invention, but not to limit the present invention. Although the present invention is described in detail with reference to the foregoing embodiments, persons of ordinary skill in the art should understand that they may still make modifications to the technical solutions described in the foregoing embodiments or make equivalent replacements to some or all technical features thereof, without departing from the scope of the technical solutions of embodiments of the present invention.

## Claims

1. A method for generating an arteriosclerosis detection interface, wherein the method comprises:
obtaining a type of an arteriosclerosis detector in use;
generating a detection interface and determining an artery detection position corresponding to the type of the arteriosclerosis detector; and
displaying the artery detection position on the detection interface.

2. The method according to claim 1, wherein
the detection interface comprises a human artery distribution diagram, and the displaying the artery detection position on the detection interface comprises: highlighting an artery segment at the artery detection position in the human artery distribution diagram.

3. The method according to claim 2, wherein
before the generating a detection interface and determining an artery detection position corresponding to the type of the arteriosclerosis detector, the method further comprises:
obtaining gender information of a to-be-detected user; and
determining a type of the human artery distribution diagram based on the gender information of the to-be-detected user.

4. The method according to claim 2, wherein the highlighting an artery segment at the artery detection position on the detection interface comprises:
enabling the artery segment at the artery detection position in the human artery distribution diagram to blink; or
displaying the artery segment at the artery detection position in the human artery distribution diagram, and hiding remaining artery segments.

5. The method according to claim 2, wherein the method further comprises:
obtaining a detection result from the arteriosclerosis detector; and
generating a detection result interface based on the detection result, wherein
the detection result interface comprises the human artery distribution diagram, and the detection result pops up at the artery detection position in the human artery distribution diagram.

6. The method according to claim 2, wherein the method further comprises:
after the detection result pops up, generating prompt information for viewing an aortic arteriosclerosis degree at a heart position in the human artery distribution diagram; and
popping up aortic arteriosclerosis degree information at the heart position in the human artery distribution diagram according to a tap-to-view instruction of a user, wherein
the aortic arteriosclerosis degree information comprises an aortic pulse wave velocity and an aortic condition analysis result.

7. The method according to claim 1, wherein the method further comprises:
generating prompt information for viewing personal information of a user; and
expanding and displaying the personal information of the user according to a tap-to-view instruction of the user, wherein
the personal information of the user comprises one or more of the following:
name, gender, age, contact information, and historical medical treatment data.

8. An apparatus for generating an arteriosclerosis detection interface, wherein the apparatus comprises:
a processor and a memory, wherein the memory is configured to store at least one instruction, and when the instruction is loaded and executed by the processor, the method for generating an arteriosclerosis detection interface according to any one of claims 1 to 7 is implemented.

9. A terminal, wherein the terminal comprises the apparatus for generating an arteriosclerosis detection interface according to claim 8.

10. An arteriosclerosis detection system, wherein the system comprises:
an arteriosclerosis detector, configured to detect a pulse wave velocity of a user; and
the terminal according to claim 9.

11. The system according to claim 10, wherein the arteriosclerosis detector comprises one or more of the following:
a smart watch, a smart band, and a body fat scale.
